# EUROPEAN PATENT APPLICATION

(11) **EP 3 061 825 A1**
(43) Date of publication of application: **31.08.2016**
(21) Application number: 14855992.5
(22) Date of filing: 24.10.2014
(51) Int. Cl.: C12N 15/12, C12N 5/074

(54) **METHOD FOR PRODUCING MEGAKARYOCYTES AND PLATELETS**

(30) Priority: 24.10.2013 ES 201331568
(71) Applicant: Fundación Pública Andaluza Progreso Y Salud, 41092 Sevilla (ES)
(72) Inventor: REAL LUNA, Pedro José, E-41092 Sevilla (ES); GARCÍA TOSCANO, Miguel, E-41092 Sevilla (ES); MENÉNDEZ BUJAN, Pablo, E-41092 Sevilla (ES); MARTÍN MOLINA, Francisco, E-41092 Sevilla (ES); NAVARRO MONTERO, Óscar, E-41092 Sevilla (ES); AYLLÓN CASES, Verónica, E-41092 Sevilla (ES); RAMOS MEJÍA, Verónica, E-41092 Sevilla (ES); BUENO UROZ, Clara, E-41092 Sevilla (ES); COBO PULIDO, Marién, E-41092 Sevilla (ES); ROMERO ESCOBAR, Tamara, E-41092 Sevilla (ES)
(74) Representative: Hoffmann Eitle
(86) International application number: PCT/ES2014/070805
(87) International publication number: WO 2015/059339

(57) **Abstract**

The present invention is based on the surprising discovery that the overexpression of the SCL gene, having nucleotide sequence SEQ ID NO: 1, increases the production of megakaryocytes and platelets from stem cells. It has therefore been proven that this increase also occurs using PSCs which, unlike CD34+ HSC cells which have limited expansion capacity, are characterized by an unlimited growth capacity, and can result in the unlimited production of platelets for clinical use.

## Description

### FIELD OF THE INVENTION

The present invention is comprised within the field of biology and medicine, and more specifically in the advanced therapy sector. Specifically, the present invention relates to the use of stem cells for producing functional platelet-producing megakaryocytes, for use against diseases derived from hematopoietic abnormalities. The present invention also relates to megakaryocytes and platelets produced by said method, and to systems and/or devices for producing same.

### BACKGROUND OF THE INVENTION

Platelets are enucleated cytoplasmic fragments that are released from mature megakaryocytes (MKs). MKs originate from hematopoietic stem cells (HSCs) through a differentiation process known as megakaryopoiesis. Once formed, MKs undergo a maturation process (thrombopoiesis) resulting in the production and release of platelets (Kaushansky, K. 2008. Blood 111(3):981-6). Platelet transfusion is necessary for the treatment of thrombocytopenias of physiological origin, leukemias, genetic disorders or disorders induced by aggressive chemotherapy. Due to the fact that the half-life of platelets is very short and that the number of platelets obtained from donors in blood banks is limited, the generation of alterative *ex vivo* systems for the production of platelets is a very promising option (Lambert, M.P. et al., 2013. Blood 121(17):3319-24.; Slichter, S.J. 2004. Transfusion Medicine Reviews 18(3):153-67.). One of the challenges in medicine is to provide hospitals with a constant supply of platelets, although it has been impossible up until now, because it depends completely on the availability and generosity of donors. Therefore, the generation of a donor-independent system capable of providing platelets for transfusion constantly and efficiently would be ideal to fulfill the needs for platelets for clinical treatment of thrombocytopenias (Lambert et al., M.P. 2013. Blood 121(17):3319-24.).

In the last two decades, several groups have been able to generate MKs and platelets *in vitro* using CD34+ HSCs obtained from peripheral blood, umbilical cord, fetal liver and bone marrow, but the limited expansion capacity of CD34+ HSC cells restricts their use as an efficient and constant source of platelets (Choi, E.S. et al. 1995. Blood 85(2):402-13; Tao, H. et al., 1999. Exp. Hematol. 27(2):293-301; Ma, D.C. et al. 2000. Eur. J. Haematol. 64(5):304-14; De Bruyn, C. et al. 2005. Stem Cells Dev. 14(4):415-24). In contrast, human embryonic stem cells (hESCs) and induced pluripotent stem cells (iPSCs) are characterized by an unlimited growth capacity and the ability to differentiate into any cell lineage, making them a very promising alternative for therapies in hematology (Thomson, J.A. et al. 1998. Science 282(5391):1145-7; Takahashi, K. et al. 2007. Cell 131(5):861-72; Krimbel, E.A. and Lu, S.J. 2011. Stem Cells Int 2011:273076.). Based on these properties, several laboratories around the world have optimized different strategies for generating functional platelets and megakaryocytes using human pluripotent stem cells (hPSCs) (Takayama, N. et al. 2008. Blood 111(11):5298-306; Takayama, N. et al. 2010. J. Exp. Med. 207(13):2817-30; Lu, S.J. et al., 2011. Cell Res. 21(3):530-45; Pick, M. et al., 2013. PLoS One 8(2):e55530). Up until now, these studies have generated megakaryocytes and platelets that can be activated *in vitro* by conventional stimuli (ADP, fibrinogen or thrombin) (Takayama, N. et al. 2008. Blood 111(11):5298-306; Lu, S.J. et al., 2011. Cell Res. 21(3):530-45) and functional platelets participating in clot formation *in vivo* (Takayama, N. et al. 2010. J. Exp. Med. 207(13):2817-30; Lu, S.J. et al., 2011. Cell Res. 21(3):530-45). However, these cells have the limitation of producing a limited number of platelets and of megakaryocytes per starting pluripotent stem cell compared with *in vivo* productions (Tijssen, M.R. and Ghevaert C.J., 2013. Thromb Haemost 11(4):593-604). Therefore, work must still be done in optimizing more efficient protocols for megakaryopoiesis and thrombopoiesis from hPSCs.

These improvements could be based on the knowledge of molecular mechanisms controlling megakaryocyte differentiation. A large number of transcription factors have been associated with megakaryopoiesis and thrombopoiesis regulation (Goldfarb, A.N. 2007. Oncogene 26(47):6795-802). Among these mechanisms, SCL/TAL1 has been the focus of attention not only in establishing hematopoiesis in mice (Shivdasani, R.A. et al., 1995. Nature 373(6513):432-434; Porcher, C. et al., 1996. Cell 86(1):47-57), but also in erythroids and megakaryocytes (Mikkola, H.K. et al., 2003. Science 421(6922):547-51; Hall, M.A. et al., 2003. PNAS 100(3):992-7; Schlaeger et al., 2005. Blood 105(10):3871-4).

It has been demonstrated that the overexpression of SCL increases hematopoietic differentiation from hPSCs and enhances the characteristics of erythroids (Yung, S. et al., 2011. Hum. Mol. Gene. 20(24):4932-46; Real, P.J. et al., 2012. Mol. Therapy 20(7):1443-53). SCL participates in the task of megakaryocytes, it is proposed that the overexpression of this hematopoietic transcription factor will increase the efficiency of megakaryopoiesis and thrombopoiesis from hPSCs. This overexpression *a priori* would not entail any risk when the platelets are transfused because the platelets are enucleated and do not carry more copies of the overexpressed genes or of the translated protein. This represents a breakthrough in the way of large-scale generation of platelets for use in transfusion in humans.

Therefore, hPSCs are an ideal tool for optimizing the protocols for human megakaryopoiesis and thrombopoiesis intended for the unlimited production of platelets for clinical use. These optimized protocols will become a platform for the study of molecular and cellular processes involved in human megakaryopoiesis and thrombopoiesis, and could be used for modeling human diseases affecting these differentiation processes, such as acute megakaryoblastic leukemias (AMKL).

### BRIEF DESCRIPTION OF THE INVENTION

A first aspect of the invention relates to the use of the *SCL*/*TAL1* gene, having nucleotide sequence SEQ ID NO: 1, hereinafter nucleotide sequence of the invention, for producing functional platelet-producing megakaryocytes from stem cells. In a preferred embodiment of this aspect, said stem cells were not produced by methods using human embryos as raw material or destroying same.

A second aspect of the invention relates to the use of a DNA or RNA gene construct, hereinafter gene construct of the invention, comprising one of the following types of sequences:
a. nucleotide sequence comprising at least the nucleotide sequence of the invention, for *in vitro* or *in vivo* transcription, or
b. nucleotide sequence corresponding to a gene expression vector or system comprising nucleotide sequence of the invention, operatively linked with at least one promoter directing the transcription of said nucleotide sequence, and with other sequences necessary or suitable for transcription and for the suitable regulation thereof in time and place;
for producing functional platelet-producing megakaryocytes from stem cells. The gene construct is preferably a vector, hereinafter vector of the invention, and even more preferably a lentiviral vector. In another preferred embodiment, the stem cells are selected from the list consisting of hematopoietic stem cells, pluripotent adult stem cells, preferably when these were not produced by methods using human embryos as raw material or destroying same, induced pluripotent stem cells, embryonic stem cells, preferably when these were not produced by methods using human embryos as raw material or destroying same, fibroblasts, induced megakaryocytes (Ono, Y. et al., 2012 Blood 120(18):3812-21) or any of the combinations thereof. They are even more preferably pluripotent stem cells (PSCs), more preferably stem cells from a mammal, and yet more preferably, human cells, and still more preferably when none of these cells were produced by methods using human embryos as raw material or destroying same.

A third aspect of the invention relates to a functional platelet-producing megakaryocyte, hereinafter megakaryocyte of the invention, produced by a method, (hereinafter "method for producing platelets of the invention"), which comprises:
a) contacting the gene construct or the vector of the invention with a stem cell,
b) selecting from step (a) stem cells which have been transduced or transformed with the gene construct and/or the vector, and
c) differentiating the stem cells of step (b) into mature megakaryocytes.

In another preferred embodiment, the stem cells are selected from the list consisting of hematopoietic stem cells, pluripotent stem cells, induced pluripotent stem cells, embryonic stem cells, preferably provided that these cells were not produced by methods using human embryos as raw material or destroying same, fibroblasts, induced megakaryocytes (Ono, Y. et al., 2012 Blood 120(18):3812-21) or any of the combinations thereof. They are even more preferably pluripotent stem cells (PSCs), more preferably stem cells from a mammal, and yet more preferably human cells, preferably provided that none of these cells were produced by methods using human embryos as raw material or destroying same. In another preferred embodiment of this aspect of the invention, the differentiation of the stem cells into megakaryocytes of step (c) is carried out by differentiating the stem cells into megakaryocyte progenitors by means of adding a composition comprising a histone deacetylase inhibitor. Even more preferably, the histone deacetylase inhibitor is Trichostatin A. In a more preferred embodiment of the invention, the differentiation of the stem cells into mature megakaryocytes of step (c) comprises co-culturing the stem cells with stromal cells.

A fourth aspect of the invention relates to a method for the production of platelets *in vitro,* hereinafter method for producing platelets of the invention, which comprises culturing the functional platelet-producing megakaryocyte as defined in the third aspect of the invention in a suitable culture medium.

A fifth aspect of the invention relates to a platelet, hereinafter platelet of the invention, produced by the method for producing platelets of the invention.

A sixth aspect of the invention relates to the use of a megakaryocyte or a platelet of the invention, in the preparation of a medicinal product.

A seventh aspect of the invention relates to the use of a megakaryocyte or a platelet of the invention, in the preparation of a medicinal product for the treatment of vascular damage.

An eighth aspect of the invention relates to the use of a megakaryocyte or a platelet of the invention, in the preparation of a medicinal product for the treatment of platelet-related pathologies and/or acute megakaryoblastic leukemias.

A ninth aspect of the invention relates to the use of a histone deacetylase inhibitor for differentiating a stem cell into megakaryocyte. In a preferred embodiment of this aspect of the invention, the histone deacetylase inhibitor is Trichostatin A.

A tenth aspect of the invention relates to a system or device for producing platelets for the *in vitro* and/or *ex vivo* production of the platelets of the invention comprising:
a) a bioreactor for the expansion of stem cells, preferably when these were not produced by methods using human embryos as raw material or destroying same, in the presence of a first growth medium in fluid communication with;
b) a maturation chamber comprising stromal cells and/or an artificial bone marrow niche and a second growth medium, in which the maturation chamber is in fluid communication with;
c) a cell separation chamber for the selection of mature megakaryocytes which is in fluid communication with;
d) a platelet production module comprising a plurality of platelet production chambers, a three-dimensional matrix, a third growth medium, and a plurality of pumps for moving one third of the growth medium through the platelet production chambers, in which the platelet production module is in fluid communication with ;
e) a platelet collection chamber.
where the stem cells of step (a) are transfected with the SCL gene. In a preferred embodiment of this aspect of the invention, the system or device for producing platelets incorporates in one or more growth media used in one of the steps thereof a histone deacetylase inhibitor. The histone deacetylase inhibitor is even more preferably Trichostatin A.

### BRIEF DESCRIPTION OF THE DRAWINGS

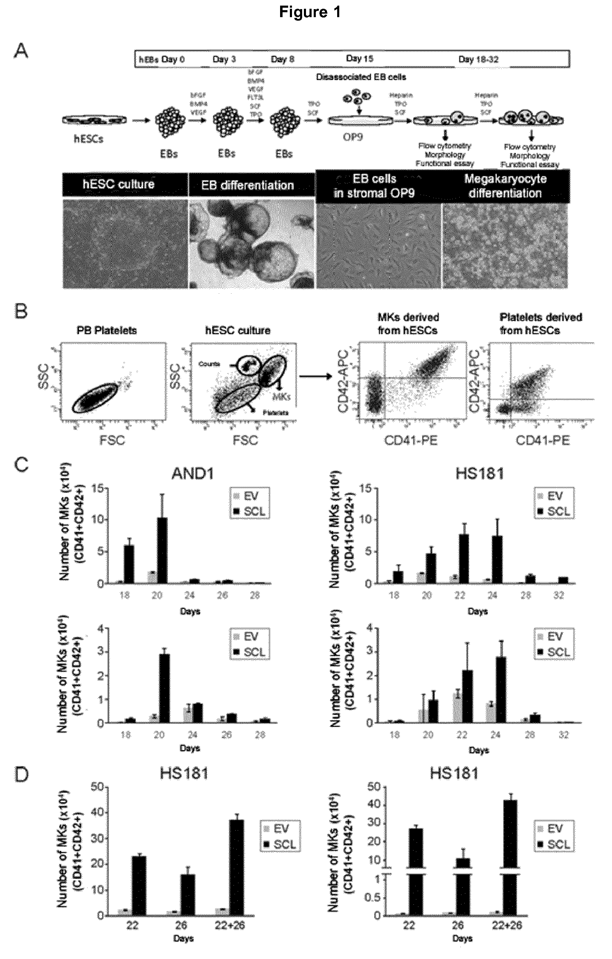

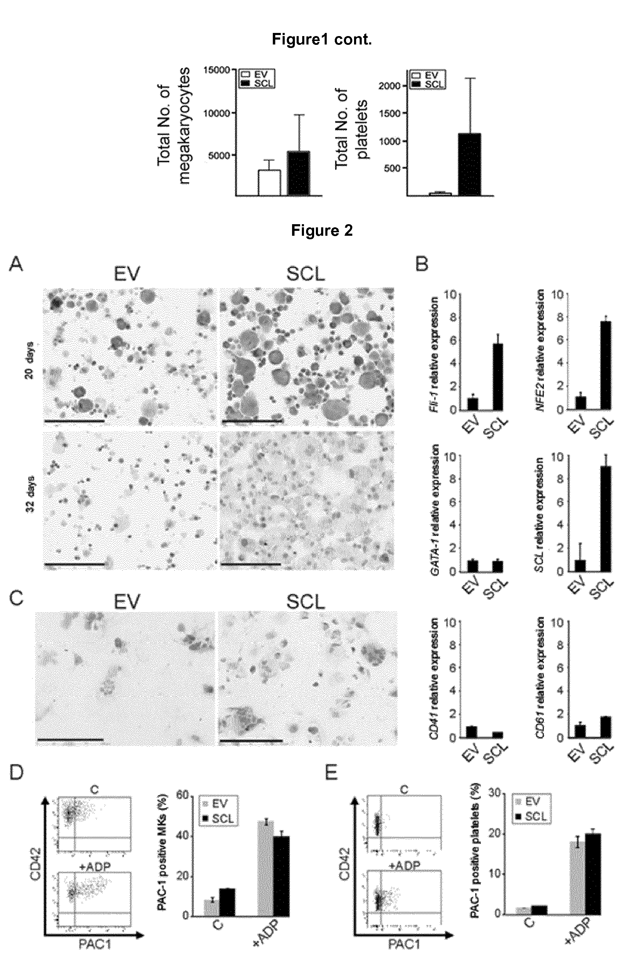

Figure 1 shows the overexpression of SCL increases the production of megakaryocytes and platelets in PSCs. A) Diagram showing the differentiation of megakaryocytes from hPSCs. This differentiation protocol is divided into two stages: EB differentiation stage (from day 0 to day 15) in the presence of hematopoietic cytokines (bFGF, VEGF, BMP4, Flt3L, SCF and TPO) and with co-culture with OP9 in the differentiation stage (from day 15 to day 32) where hPSCs originate from the growth along with OP9 stromal cells supplemented with heparin, TPO and SCF. B) Flow cytometry charts showing how the megakaryocytic cells (MKs) and platelets are identified and analyzed during the OP9 co-culture differentiation stage. Peripheral blood (PB) platelets were obtained from blood bank donors for establishing the configuration of the flow cytometry analysis. C) The kinetics of emergence of megakaryocytes and platelets from two different lines (AND1 and HS181) transduced with an empty vector (EV) and with the vector expressing *SCL* (*SCL*)*.* The top panels depict the number of megakaryocytes (CD41+ CD42+). The bottom panels depict the number of platelets (CD41+ CD42+). D) The kinetics of accumulation of megakaryocytes and platelets of HS181 transduced with the empty vector (EV) or with the vector overexpressing SCL, on days 22 and 26 days of megakaryocytic development. The data represents the mean ± SEM of 2-3 independent experiments using the HS181 hPSC line. E) The overexpression of SCL increases the production of megakaryocytes and platelets using iPSCs. Depiction of the total megakaryocyte and platelet production throughout megakaryocyte differentiation *in vitro* in control cells (EV) or cells transduced with SCL (SCL). The data represents the mean ± standard error of the mean of 3 independent experiments.
Figure 2 shows the morphological, molecular and functional characterization of megakaryocytes and platelets derived from HPSCs. A) Papanicolaou staining of cells purified from the supernatant of differentiated HS181 EV and SCL cells of co-cultures after 20 and 32 days. B) Quantitative analysis using RT-PCR of several transcription factors of (FLI-1, NFE2, GATA-1 and SCL) and surface markers (CD41 and CD61) expressed in cell derivatives collected from HS181 EV and SCL culture supernatants after 22 days of megakaryocyte differentiation. The relative expression is shown normalized to the EV cells. The data represents the mean ± SEM of 2 independent experiments. C) Papanicolaou staining of megakaryocytes derived from HS181 EV or SCL which are activated by thrombin (2 U/ml) for 2 hours. D) Flow cytometry analysis of the megakaryocytic control (C) or ADP-activated cells (+ADP) on day 26 of megakaryocyte differentiation. The PAC1 antibody was used to detect the activated form of integrin αIIbβ3 after treatment with ADP. The data represents the mean ± SEM of 2 independent experiments.

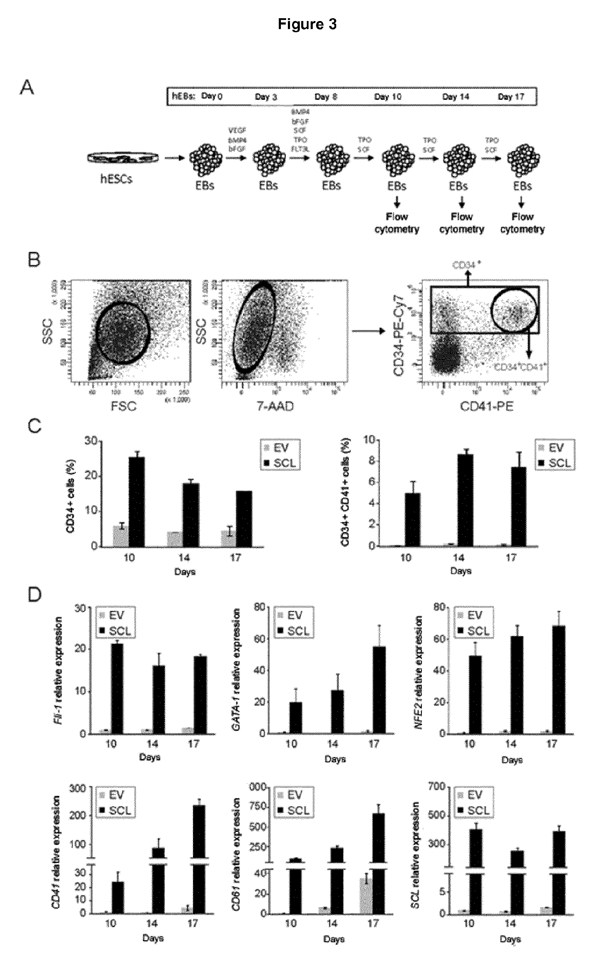

Figure 3 shows that the overexpression of SCL accelerates the emergence of megakaryocyte progenitors from PSC. A) Schematic depiction of the analysis of megakaryocyte progenitors from PSCs. This differentiation protocol is reduced to EB differentiation stage (from day 0 to day 8) in the presence of hematopoietic cytokines (bFGF, VEGF BMP4, FIt3L, SCF and TPO) and TPO and SCL (from day 8 to day 17). Flow cytometry analysis was performed on days 10, 14 and 17 of megakaryocyte differentiation. B) Graphs representative of flow cytometries showing how megakaryocyte progenitors (CD34+ and CD34+ CD41+) are identified and analyzed. C) The kinetics of emergence of megakaryocyte progenitors (CD34+ and CD34+ CD41+) from the AND1 PSC cell line transduced with the empty vector (EV) or expressed SCL vector (SCL). The data represents the mean ± SEM of 2 independent experiments. D) RT-PCR quantitative analysis of several transcription factors associated with megakaryocytic development (*FIi-1, NFE2, GATA-1* and SCL) and surface markers (*CD41* and *CD61*) expressed in EBs of AND1 EV and SCL cultures on days 10, 14 and 17 of megakaryocyte differentiation. The relative expression is shown normalized to EBs from AND1 EV on day 10 of the differentiation. The data represents the mean ± SEM of 2 independent experiments.

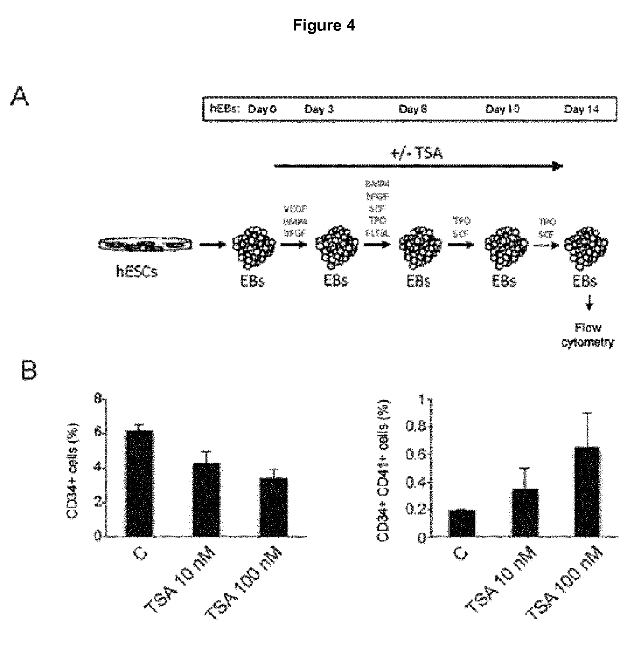

Figure 4 shows that Trichostatin A accelerates the emergence of megakaryocyte progenitors from PSCs. A) Diagram for the analysis of megakaryocyte progenitors from PSCs in the presence or absence of Trichostatin A (TSA). Flow cytometry analysis was developed on day 14 of megakaryocyte differentiation. B) Analysis of the emergence of megakaryocyte progenitors (CD34+ and CD34+ CD41+) from wild-type AND1 PSCs at two different concentrations (10 and 100 nM). The data represents the mean ± SEM of 2 independent experiments.

### DETAILED DESCRIPTION OF THE INVENTION

The authors of the present invention have demonstrated that the overexpression of the SCL gene, having nucleotide sequence SEQ ID NO: 1, increases the production of megakaryocytes and platelets from stem cells. Furthermore, they have been proven that this increase also occurs using PSCs which, unlike CD34+ HSC cells which have limited expansion capacity, are characterized by an unlimited growth capacity, and can result in the unlimited production of platelets for clinical use.

Therefore, a first aspect of the invention relates to the use of the SCL gene, having nucleotide sequence (GenBank NM_003189.2, NP_003180.1) SEQ ID NO: 1, hereinafter nucleotide sequence of the invention, for producing functional platelet-producing megakaryocytes from stem cells. Use of the SCL gene for producing functional platelet-producing megakaryocytes from stem cells is preferably *in vitro.*

In a preferred embodiment of this aspect of the invention, it is understood that the SCL gene has a nucleotide sequence with an identity of at least 50%, more preferably 60%, 70%, 80%, 90%, 95%, 98% and yet more preferably 99% with the sequence SEQ ID NO: 1.

It can be anticipated that the degree of identity/similarity of the homologous sequences with respect to that listed in sequence SEQ ID NO: 1 is at least 50% or greater, and more preferably at least 60%, 70%, 80%, 90%, 95%, 98% or 99%. The correspondence between the nucleotide sequence/sequences of the putative sequence/sequences and the sequence listed in SEQ ID NO: 1 can be determined using methods known in the art. Sequence comparison methods are known in the state of the art, and include, although without being limited to, the BLAST or BLASTN program, and FASTA (Altschul et al., J. Mol. Biol. 215: 403-410 (1999)).

As it is used herein, the term "homology" refers to the similarity between two structures due to a common evolutionary descent, and more specifically, to the similarity between two or more nucleotide or amino acid sequences.

As it is used herein, the term "identity", refers to the proportion of identical nucleotides or amino acids between two nucleotide or amino acid sequences being compared. Sequence comparison methods are known in the state of the art, and include, although without being limited to, the GAG program, including GAP (Devereux et al., Nucleic Acids Research 12: 287 (1984) Genetics Computer Group University of Wisconsin, Madison, (WI)); BLAST, BLASTP or BLASTN, and FASTA (Altschul et al., J. Mol. Biol. 215: 403-410 (1999)).

The SCL or *TAL1* "T-cell acute lymphocytic leukemia 1" gene, also referred to as TCL5; tal-1; bHLHa17, is located in chromosome 1 (1p32). Its amino acid sequence is in SEQ ID NO: 2, (having nucleotide sequence SEQ ID NO: 1 with GenBank accession number (NCBI) NM_003189.2).

In the context of the present invention, SCL is also defined by a nucleotide or polynucleotide sequence which constitutes the sequence encoding the protein listed in SEQ ID NO: 2 and would comprise various variants originating from:
a) nucleic acid molecules encoding a polypeptide comprising the amino acid sequence of SEQ ID NO: 2,
b) nucleic acid molecules the complementary strand of which hybridizes with the polynucleotide sequence of a),
c) nucleic acid molecules the sequence of which differs from a) and/or b) due to genetic code degeneration,
d) nucleic acid molecules encoding a polypeptide comprising the amino acid sequence with an identity of at least 80%, 90%, 95%, 98% or 99% with SEQ ID NO: 2, and in which the polypeptide encoded by said nucleic acids has the activity and structural characteristics of SCL.

A second aspect of the invention relates to the use of a DNA or RNA gene construct, hereinafter gene construct of the invention, comprising one of the following types of sequences:
a. nucleotide sequence comprising at least the nucleotide sequence of the invention, according to any of the aforementioned definitions, for *in vitro* or *in vivo* transcription, or
b. nucleotide sequence corresponding to a gene expression vector or system comprising the nucleotide sequence of the invention, according to any of the aforementioned definitions, operatively linked with at least one promoter directing the transcription of said nucleotide sequence, and with other necessary or suitable sequences for transcription and for the suitable regulation thereof in time and place;
   for producing functional platelet-producing megakaryocytes from stem cells. The gene construct is preferably used *in vitro.* In another preferred embodiment, the gene construct is a vector, hereinafter vector of the invention, and even more preferably a lentiviral vector. In another preferred embodiment, the stem cells are selected from the list consisting of hematopoietic stem cells, pluripotent stem cells, induced pluripotent stem cells, embryonic stem cells, fibroblasts, induced megakaryocytes (Ono, Y. et al., 2012 Blood 120(18):3812-21) or any of the combinations thereof. They are even more preferably pluripotent stem cells (PSCs), more preferably stem cells from a mammal, and yet more preferably human cells.

In the context of the present invention, the stem cells referred to throughout the text are preferably characterized by not having been produced by methods using human embryos as raw material or destroying same. More preferably, the stem cells are pluripotent stem cells, and even more preferably, the stem cells are selected from induced pluripotent stem cells, a hESC produced without the destruction and more preferably without the use of an embryo, a cell produced by somatic cell nuclear transfer (SCNT), a stem cell produced by parthenogenesis, a cell produced by transdifferentiation, or a progenitor stem cell.

A large number of these constructs, expression vectors or systems can be produced by conventional methods known by the persons skilled in the art and form part of the present invention.

A "vector" is a replicon or an integrative vector to which another polynucleotide segment has been bound to carry out the replication and/or expression of the bound segment.

A "replicon" is any genetic element that behaves like an autonomous polynucleotide replication unit within a cell; i.e., it is capable of replicating under its own control.

An integrative vector is any genetic element which integrates itself in the cell genome and remains stable therein.

A "lentiviral vector" is an integrative vector derived from lentivirus (non-oncogenic retrovirus capable of infecting all type of cells, both quiescent cells and dividing cells). Lentiviral vectors can insert DNA fragments of intermediate size which can reach up to 8000 bp. The main drawback of these vectors is that their integration into the host cell genome is completely random. Today, most of these vectors are derived from human immunodeficiency virus (HIV).

"Control sequence" refers to polynucleotide sequences which are necessary to carry out the expression of the sequences to which they are ligated. The nature of said control sequences differs depending on the host organism; in prokaryotes, said control sequences generally include a promoter, a ribosomal binding site, and termination signals; in eukaryotes, said control sequences generally include promoters, termination signals, enhancers and, sometimes, silencers. It is envisaged that the term "control sequences" includes at least all the components the presence of which is necessary for the expression, and it can also include additional components the presence of which is advantageous.

As used herein, the term "promoter" refers to a DNA region located upstream of the transcription start point, and in particular, capable of initiating transcription in a plant cell, whether or not the promoter is of plant origin. There are promoters which initiate transcription in a specific type of tissues and they are referred to as "tissue-specific" promoters. An "inducible" promoter or a "repressible" promoter is a promoter that is under the control of the environment. Tissue-specific promoters, tissue-preferred promoters, cell type-specific promoters, or inducible promoters are types constituting the class of "non-constitutive" promoters. A "constitutive" promoter is a promoter which is active under most environmental conditions.

"Operatively linked" refers to a juxtaposition in which the components thus described have a relationship that allows them to function in the intended manner. A control sequence "operatively linked" to a sequence which is transcribed into the nucleotide sequence of the invention is ligated such that the expression of the coding sequence is achieved in conditions compatible with the control sequences.

A "coding sequence" is a polynucleotide sequence which is transcribed into an mRNA and/or translated into a polypeptide when it is under the control of suitable regulatory sequences. The limits of the coding sequence are determined by means of a translation start codon at the 5' end and a translation termination codon at the 3' end. A coding sequence can include, but is not limited to, mRNA, cDNA, and recombinant polynucleotide sequences.

The terms "polynucleotide" and "nucleic acid" are used interchangeably to refer to polymeric forms of nucleotides of any length, both ribonucleotides (RNA) and deoxyribonucleotides (DNA).

The terms "amino acid sequence", "peptide", "oligopeptide", "polypeptide" and "protein" are used interchangeably to refer to a polymeric form of amino acids of any length, which may or may not be chemically or biochemically modified.

### MEGAKARYOCYTES OF THE INVENTION

A third aspect of the invention relates to a functional platelet-producing megakaryocyte, hereinafter megakaryocyte of the invention, produced by a method which comprises:
a) contacting the gene construct or the vector of the invention with a stem cell,
b) selecting from step (a) stem cells which have been transduced or transformed with the gene construct and/or the vector, and
c) differentiating the stem cells of step (b) into mature megakaryocytes.

In another preferred embodiment, the stem cells are selected from the list consisting of hematopoietic stem cells, pluripotent stem cells, induced pluripotent stem cells, embryonic stem cells, fibroblasts, induced megakaryocytes (Ono, Y. et al., 2012 Blood 120(18):3812-21), megakaryocyte progenitor cells or any of the combinations thereof. They are even more preferably pluripotent stem cells (PSCs), more preferably stem cells from a mammal, and yet more preferably human cells.

hESCs are pluripotent stem cells derived from the internal cell mass of an embryo in the initial phase and are capable of differentiating into all derivatives of the three primary germinal layers: ectoderm, endoderm and mesoderm. These cells are capable of differentiating into all types of cells in the human body. It must be pointed out that these cells have been used for the purpose of illustrating the present invention.

iPSCs are a type of pluripotent stem cell artificially derived from a mature cell. Typically, adult somatic cells are induced to become pluripotent cells by means of activating specific immaturity genes in these cells.

Hematopoietic stem cells are progenitor cells circulating in blood and residing in the bone marrow and have the potential to give rise to all hematopoietic cells. Hematopoietic stem cells can be acquired from bone marrow, peripheral blood with apheresis machines, or from umbilical cord or placenta after birth.

As it is used herein, the term "megakaryocyte progenitor cells" refers to compromised hematopoietic stem cells with at least the megakaryocyte line and includes, but are not limited to, blood cells from the umbilical cord, bone marrow and peripheral blood, as well as hematopoietic stem cells, embryonic stem cells, including human embryonic stem cells, and induced pluripotent stem cells.

For the purposes of the present description, the terms "stem cells" and "megakaryocyte progenitor cells" are interchangeable and refer to pluripotent, multipotent or unipotent stem cells or progenitor cells which are capable of differentiating into megakaryocytes and have the potential to produce platelets. Additionally, the terms "stem cells" and "megakaryocyte progenitor cells" preferably refer to cells which were not produced by methods using human embryos as raw material or destroying same.

For the purposes of the present description, the term "growth factors" refers to proteins and other non-protein factors supporting cell growth, maintenance, maturation and differentiation.

For the purposes of the present description, the term "growth medium" refers to liquid or semi-solid aqueous medium including electrolytes, energy sources, growth factors and other materials necessary for culturing cells *ex vivo.*

Megakaryocytes have the notable characteristic of duplicating their nuclear and cell contents without cell division through a process called endomitosis. Through endomitosis, the megakaryocyte grows to a large size and can reach more than 64 times normal nuclear contents. The increase in nuclear contents, or polyploidy, plays a fundamental role in platelet formation allowing the cell to produce large amounts of proteins and organelles necessary for platelet formation and function. It is important to point out that mature megakaryocytes have also large amounts of extra cell membrane with which to produce platelets. Polyploidization can be induced using only reagents or reagents in different combinations known in the state of the art. The authors of the present invention have demonstrated that the use of histone deacetylase inhibitors induces megakaryocyte progenitor maturation.

Therefore, in another preferred embodiment of this aspect of the invention, the differentiation of the stem cells into megakaryocytes of step (c) is carried out by differentiating the stem cells into megakaryocyte progenitors by means of adding a composition comprising a histone deacetylase inhibitor. The histone deacetylase inhibitor is even more preferably Trichostatin A. In a more preferred embodiment of the invention, the differentiation of the stem cells into mature megakaryocytes of step (c) comprises co-culturing the stem cells with stromal cells.

Histone deacetylases (or HDACs) are a type of enzymes involved in the elimination of acetyl groups from lysine residues in histones. There is a long history of using histone deacetylase inhibitors (HDIs), such as valproic acid, as drugs in psychiatry and neurology as they have stabilizing and anti-seizure action. More recently, HDIs have been used as mitigating agents in the treatment of neurodegenerative diseases. Significant effort has also been made in recent years in the development of HDIs for cancer therapies, such vorinostat, which has been recently approved for the treatment of cutaneous T-cell lymphoma (CTCL).

The megakaryocytes of the invention can also form part of a population of megakaryocytes. Therefore, another aspect of the invention relates to an isolated cell population, hereinafter cell population of the invention, comprising at least one megakaryocyte of the invention. In a preferred embodiment, the cell population of the invention comprises at least 20%, preferably 40%, and even more preferably 50%, 60%, 80%, 90%, 95%, or 99% of megakaryocytes of the invention.

The megakaryocyte of the invention is preferably an isolated megakaryocyte. The term "isolated" indicates that the cell (megakaryocyte) or the population of megakaryocytes of the invention to which reference is made are not found in their natural environment. In other words, the cell or cell population has been separated from its surrounding tissue. It particularly means that said cell or cell population is substantially free of other cells normally present in blood or in a hemoderivative thereof, of a subject from whom blood has been withdrawn for isolating said cell or cell population. It also refers to cells or cell populations which have been isolated from the organism in which they originate. The term also includes cells which have been isolated from of an organism and reintroduced into the same organism, or into another organism.

### METHOD FOR PRODUCING PLATELETS OF THE INVENTION

A fourth aspect of the invention relates to a method for the production of platelets *in vitro,* hereinafter method for producing platelets of the invention, which comprises culturing the functional platelet-producing megakaryocyte of the invention in a suitable culture medium.

Suitable culture media are known in the state of the art, such as for example, but without limitation, those described in Takayama, N. et al. 2008. Blood 111(11):5298-306; Takayama, N. et al. 2010. J. Exp. Med. 207(13):2817-30; Lu, S.J. et al., 2011. Cell Res. 21(3):530-45; Ono, Y. et al., 2012 Blood 120(18):3812-21; Pick, M. et al., 2013. PLoS One 8(2):e55530.

A fifth aspect of the invention relates to a platelet, hereinafter platelet of the invention, produced by the method for producing platelets of the invention.

### USES OF THE MEGAKARYOCYTES AND PLATELETS OF THE INVENTION

A sixth aspect of the invention relates to the use of a megakaryocyte or a platelet of the invention, in the preparation of a medicinal product.

A seventh aspect of the invention relates to the use of a megakaryocyte or a platelet of the invention, in the preparation of a medicinal product for the treatment of vascular damage.

An eighth aspect of the invention relates to the use of a megakaryocyte or a platelet of the invention, in the preparation of a medicinal product for the treatment of platelet-related pathologies.

Platelet-related pathologies are known in the state of the art, for example among others, but without limitation, thrombocytopenias of physiological origin, leukemias, genetic disorders or disorders induced by aggressive chemotherapy, Glanzmann's thrombasthenia or Bernard-Soulier syndrome and/or acute megakaryoblastic leukemias.

Both the megakaryocytes and the platelets of the invention can form part of a composition, hereinafter first composition of the invention. In a preferred embodiment, the composition is a pharmaceutical composition (hereinafter pharmaceutical composition of the invention), and more preferably, said composition can incorporate other active ingredient/ingredients. In a preferred embodiment, the pharmaceutical composition of the invention further comprises a pharmaceutically acceptable vehicle. In another preferred embodiment, the pharmaceutical composition of the invention further comprises another active ingredient.
The term "pharmaceutically acceptable vehicle" refers to a vehicle that must be approved by a regulatory agency of the federal government or state government or listed in the United States Pharmacopeia or the European Pharmacopeia, or another Pharmacopeia generally recognized for use in animals, and more specifically in humans.

The term "vehicle" refers to a diluent, adjuvant, excipient or carrier which must be used to administer the megakaryocytes or the population of megakaryocytes of the invention or said composition comprising stem cells of the invention which can be produced according to the method of the invention; obviously, said vehicle must be compatible with said cells. Non-limiting, illustrative examples of said vehicle include any physiologically compatible vehicle, for example, isotonic solutions (e.g., 0.9% NaCl sterile saline solution, phosphate buffered saline (PBS) solution, Ringer-lactate solution, etc.), optionally supplemented with serum, preferably with autologous serum; cell culture media (e.g., DMEM, etc.); or, alternatively, a solid, semisolid, gelatinous or viscous support medium, such as collagen, collagen-glycosamino-glycan, fibrin, polyvinyl chloride, polyamino acids, such as polylysine, or polyornithine, hydrogels, agarose, silicone dextran sulfate. Likewise, if desired, the support medium can contain, in specific embodiments, growth factors or other agents. If the support is solid, semisolid or gelatinous, the megakaryocytes can be introduced in a liquid phase of the vehicle which is subsequently treated such that it becomes a more solid phase.

The pharmaceutical composition of the invention, if desired, can also contain, when necessary, additives for increasing, controlling or otherwise directing the desired therapeutic effect of the megakaryocytes, which comprise said pharmaceutical composition, and/or auxiliary substances or pharmaceutically acceptable substances, such as buffering agents, surfactants, cosolvents, preservatives, etc. To stabilize cell suspension, it is also possible to add metal chelating agents. The stability of the cells in the liquid medium of the pharmaceutical composition of the invention can be improved by means of adding additional substances, such as for example, aspartic acid, glutamic acid, etc. Said pharmaceutically acceptable substances which can be used in the pharmaceutical composition of the invention are generally known by the persons skilled in the art and are normally used in the preparation of cell compositions. Examples of suitable pharmaceutical vehicles are described, for example, in "Remington's Pharmaceutical Sciences", by E.W. Martin. Additional information about said vehicles can be found in any manual of pharmaceutical technology (Galenic Pharmacy).

As it is used herein, the term "active ingredient", "active substance", "pharmaceutically active substance", or "pharmaceutically active ingredient" means any component which potentially provides a pharmacological activity or another different effect in diagnosing, curing, mitigating, treating or preventing a disease, or which affects the structure or function of the human body or the body of other animals. The term includes those components promoting a change in the preparation of a drug and are present therein in an envisaged modified form providing the specific activity or the effect.

As it is used herein, the term "medicinal product" refers to any substance used for the prevention, diagnosis, relief, treatment or cure of diseases in human beings and animals.

The pharmaceutical composition of the invention will be formulated according to the chosen dosage form. The pharmaceutical composition of the invention can be prepared in a liquid or gel dosage form, for example, in the form of a suspension, for being injected or infused into the individual. Non-limiting, illustrative examples include the formulation of the pharmaceutical composition of the invention in a sterile suspension with a pharmaceutically acceptable excipient, such as a isotonic solution, for example, phosphate buffered saline (PBS) solution, or any other suitable pharmaceutically acceptable vehicle, for the parenteral administration to a subject, e.g., a human being, preferably intravenously, intraperitoneally, subcutaneously, etc., although other alternative administration routes also are possible.

The pharmaceutical composition of the invention will be administered to the individual using conventional means. For example, said pharmaceutical composition can be administered to said individual intravenously using suitable devices, such as syringes, catheters (a standard peripheral intravenous catheter, a central venous catheter or a pulmonary artery catheter, etc.), needles, cannulas, etc. The flow of the cells can be controlled by serial inflation and deflation series of distal and proximal balloons located within the patient's vasculature, thereby creating temporary flow-free zones which promote cellular therapeutic action. In all cases, the pharmaceutical composition of the invention will be administered using equipment, apparatuses and devices suitable for the administration of cell compositions and known by the person skilled in the art.

As understood by the person skilled in the art, the direct administration of the pharmaceutical composition of the invention to the site intended for benefiting from said administration can sometimes be advantageous. Therefore, the direct administration of the pharmaceutical composition of the invention to the desired organ or tissue can be achieved by direct administration (e.g., by injection, etc.) in the outer surface of the affected organ or tissue by means of inserting a suitable device, e.g., a suitable cannula, by arterial or venous infusion (including retrograde flow mechanisms) or by other means mentioned in this description or known in the art.

The pharmaceutical composition of the invention, if desired, can be stored until the time of application by means of the conventional methods known by the persons skilled in the art. This pharmaceutical composition can also be stored together with additional medicinal products useful in the treatment of diseases, in an active form comprising a combined therapy.

### COMPOSITIONS AND CULTURE MEDIUM OF THE INVENTION

The authors of the present invention have also demonstrated that the use of a histone deacetylase inhibitor serves to enhance the differentiation of a stem cell into megakaryocyte. Said inhibitors can therefore be used to improve the *in vitro* culture conditions of megakaryocyte progenitors and immature megakaryocytes. The compositions and culture medium of the invention increase maturation rates of megakaryocyte progenitors and immature megakaryocytes which can be used to increase platelet production.

Therefore, a ninth aspect of the invention relates to the use of a histone deacetylase inhibitor for differentiating a stem cell into megakaryocyte. In a preferred embodiment of this aspect of the invention, the histone deacetylase inhibitor is Trichostatin A. Said inhibitors can also form part of a composition, hereinafter second composition of the invention. Furthermore, the inhibitors and/or the second composition of the invention can also form part of a culture medium or of a pharmaceutical composition for use thereof as a medicinal product.

### SYSTEM FOR PRODUCING PLATELETS

A tenth aspect of the invention relates to a system or device for producing platelets for the production *in vitro* and/or *ex vivo* of the platelets of the invention comprising:
a) a bioreactor for the expansion of stem cells in the presence of a first growth medium in fluid communication with;
b) a maturation chamber comprising stromal cells and/or an artificial bone marrow niche and a second growth medium, in which the maturation chamber is in fluid communication with;
c) a cell separation chamber for the selection of mature megakaryocytes which is in fluid communication with;
d) a platelet production module comprising a plurality of platelet production chambers, a three-dimensional matrix, a third growth medium, and a plurality of pumps for moving one third of the growth medium through the platelet production chambers, in which the platelet production module is in fluid communication with ;
e) a platelet collection chamber.
where the stem cells of step (a) are transfected with the SCL gene.

In a preferred embodiment, the system or device for producing platelets is used for increasing stem cell and/or megakaryocyte progenitor expansion, it therefore incorporates in one or more growth media used in one of the steps thereof a histone deacetylase inhibitor. The histone deacetylase inhibitor is even more preferably Trichostatin A. In another preferred embodiment of the invention, the stromal cells are OP9 stromal cells.

The devices for producing platelets or cell culture systems are known in the state of the art and the system is not limited to the device described in this document. Therefore, for example but without limitation, the bioreactors, vessels, chambers and bags are cell collection bags, such as sterile blood collection bags known by the person with average skill in the art of blood banks. In other embodiments, the vessels, chambers and bags are sterile biocompatible containers having any design.

### EXAMPLES OF THE INVENTION

### Material and methods

### hPSC culture

The AND1, HS181 hPSC lines, (hESCs) (empty vector (EV) or SCL (SCL)) have already been characterized (Real, P.J. et al., 2012. Mol. Therapy 20(7):1443-53). These lines were kept undifferentiated in nutrient-free culture following the protocol described by Ramos-Mejia, V. et al., 2010. Mol. Therapy 18(12):2173-81). The MUSH001 iPSC cells were donated by Dr. Cibeli of Michigan University and were transduced with EV or SCL. After selection with G418 for 15 days, stable cell lines constitutively expressing SCL were established. In summary, the hPSCs were cultured on Matrigel (BD Bioscience, San Jose, CA) in T25 flasks in a nutrient medium supplemented with 8 ng/mL of basic fibroblast growth factor (bFGF) (Milteny Biotec, Bergisch Gladbach, Germany). The medium was renewed daily and confluent cultures were separated weekly by means of collagenase IV for 2-5 minutes (Invitrogen, Edinburgh, Scotland). National ethics committee approval was obtained for studies with embryos for working with hPSCs.

### Megakaryocytes differentiation from PSCs (hESCs and PSCs

The megakaryocyte differentiation protocol was adapted from Dr. Lanza's group (Lu, S.J. et al., 2011. Cell Res. 21(3):530-45). In summary, undifferentiated, confluent hPSCs in T-25 devices were treated with collagenase IV for 2-5 minutes and excess Matrigel was discarded. For the purpose of forming embryoid bodies (EBs), the hPSCs were transferred to ultra-low attachment plates (Corning, Lowell, MA) in EB medium (KO-Dulbecco's modified Eagle's medium supplemented with 20% fetal bovine serum not inactivated by heat, 1% non-essential amino acids, 1 mmol/L of L-glutamine and 0.1 mmol/L of 3-mercaptoethanol) and without cytokines. The medium was changed the next day to eliminate cell debris and the EB medium supplemented with bone morphogenetic protein 4 (BMP4) (50 ng/mL), vascular endothelial growth factor (VEGF) (50 ng/mL) and fibroblast growth factor (bFGF) (20 ng/mL) was refreshed. EB medium containing BMP4 (50 ng/mL), VEGF (50 ng/mL) and bFGF (20 ng/mL), stem cell factor (SCF) (20 ng/mL), thrombopoietin (TPO) (20 ng/mL), FMS-like tyrosine kinase 3 ligand (FLT3L) (20 ng/mL) continued to be used from day 3 to day 8. From day 8 to the end of the differentiation phase (15 days), the cytokine cocktail supplemented with EB medium was simplified with TPO (50 ng/mL) and SCF (20 ng/mL). All the cytokines were acquired from PeproTech (London, United Kingdom).

On day 10 of EB differentiation, 3x10⁴ cells per well were seeded in 6-well plates to form cell stroma. On day 15 of EB differentiation stage, the EBs were disassociated with collagenase B (Roche, Switzerland) for 2 hours at 37 °C followed by 10 minutes of incubation with cell dissociation buffer (Invitrogen) at 37°C. The cells were washed with PBS, centrifuged at 1200 rpm for 4 minutes. The single-cell suspension was obtained by means of gentle pipetting with 2 ml of OP9 differentiation medium (DM: alpha basal medium-MEM, 10% FBS not inactivated by heat, 100 µM monothioglycerol and 50 mg/mL of ascorbic acid containing TPO (100 ng/mL), SCF (50 ng/mL) and heparin (25 U/mL). The cells were cultured in DM for the rest of OP9 differentiation stage (from days 15-32) with the medium refreshed continuously every 2-3 days. From day 20 onwards, metalloproteinase inhibitor GM6001 (Merck Millipore, Bellerica, MA) (50 µM) was added to the cell culture to prevent the cleavage of CD42b into platelets.

To evaluate megakaryocyte differentiation, a CD41a and CD42b surface antigen analysis in the OP9 co-culture was carried out from day 18 until the end of the differentiation protocol every 2 days. Half of the medium was collected, picking up cells in suspension, and it was completed with the same amount of new medium to continue the differentiation culture. The cells were centrifuged at 800 g for 5 minutes, stained in PBS with 2% FCS and 2 mM of EDTA using mouse anti-human antibodies CD41a-PE (eBiosciences, San Diego, CA) (1/100 dilution) and CD42b-APC (Biolegend, San Diego, CA) (1/50 dilution) and analyzed with a flow cytometer FACSCanto II equipped with the Diva FACS software (Becton Dickinson, Franklin Lakes, NJ). The quantification of the number of cells and platelets that are positive for both CD41a⁺ and CD42b⁺ were developed by adding a known amount of Perfect-Count microspheres (Cytognos, Salamanca, Spain) to the cell suspension before FACS analysis.

### integrin αIIbβ3 with PAC-1-linked activation capacity

The megakaryocytes and platelets were collected by centrifugation at 800 g for 5 minutes, washed with Tyrode buffer (NaCl (150 mM), KCI (2.9 mM), NaHCO₃ (12 mM), glucose (0.1%), BSA (0.1%), HEPES (5 mM)) with calcium and magnesium (CaCl₂ (1 mM) and MgCl₂ (1 mM)) and resuspended in 100 µL of the same buffer. The platelets and cells were then stained with the anti-CD42b-APC antibody for 20 minutes at room temperature, activated by means of adding 70 M of 5' adenine diphosphate (ADP) for 10 minutes at room temperature and were subsequently stained using the PAC-1-FITC antibody (BD biosciences) which recognizes the activated form of integrin αIIbβ3. The cells and platelets were incubated for another 20 minutes at room temperature. The control cells were stained with both antibodies but not activated by ADP. Finally, the PAC-1 staining was analyzed using a FACSCanto II flow cytometer.

### Activation by fibrinogen

The day before the activation experiment, 8 chamber wells (Thermo Fisher Scientific, Waltham, MA) were coated with 100 g/mL of fibrinogen (Sigma-Aldrich, St Louis, MO) prepared in PBS and incubated overnight at 4°C. The platelets and cells were collected, centrifuged at 800 g for 5 minutes, resuspended in Tyrode buffer. Since they are activated with ADP (50 µM) or thrombin (2 U/mL), the megakaryocytes and platelets were incubated for 2 hours at 37°C, then fixed with 2% paraformaldehyde (PFA) for 20 minutes and washed with PSB.

### Cytochemistry

To perform cytological evaluation in the cell suspension of the OP9 co-culture, the cells were collected, fixed with 2% paraformaldehyde and washed twice with PBS. The cells were then fixed to slides treated with polylysine using cytospin at 800 g for 15 minutes, they were dried and stained using Papanicolaou staining. Alternatively, the cells and platelets with activated fibrinogen were stained using the same protocol. The stained slides were analyzed under light microscope and the images were taken with a 2.0 NanoZoomer scanner (Hamamatsu Photonics, Hamamatsu City, Japan).

### Megakaryocytes precursor analysis

The emergence of megakaryocyte precursor cells (CD34⁺ and CD34⁺ CD41a⁺) during EB differentiation stages on days 10, 14 and 17 of differentiation was evaluated. One third of the EBs growing in a 6-well plate were collected and washed once with PBS. Similarly, to evaluate the effect of Trichostatin A (TSA) on differentiation, the EBs were treated with 10 nM or 100 nM of DMSO or TSA for 14 days. In both cases, the EBs were disassociated with collagenase B and disassociation buffer as described previously. A homogenous cell suspension was obtained after washing the EBs with PBS and being carefully resuspended in 300 L of PBS with 2% FCS and 2 mM of EDTA until no clumps were observed. The disassociated cells were stained with anti-CD34-PECy7 antibodies (Miltenyi Biotec, Bergisch Gladbach, Germany) and anti-CD41a-PE antibodies and 7AAD. Living cells identified by 7AAD exclusion were analyzed using a FACSCanto II flow cytometer.

### RNA isolation, RT-PCR and qPCR analysis

The total RNA from undifferentiated hPSCs, hEBs and cell suspension of OP9 co-culture were isolated using Trizol (Invitrogen) as described previously (Real, P.J. et al., 2009. Nat. Med. 15(1):50-8). The cDNA was generated with the "Super Script First Strand Synthesis System" for RT-PCR (Invitrogen) and analyzed by means of real-time quantitative PCR (qRT-PCR) using Brilliant III Ultra-Fast SYBR Green QPCR Master Mix (Agilent Technologies, La Jolla, CA) and the QPCR Mx3005P system (Agilent Technologies). The GAPDH gene was used to normalize the data and the relative expression was calculated using the ΔΔCT method. The primer sequences are listed in Table 1:

| Primer name | Sequences | SEQ ID |
|---|---|---|
| GAPDH Fw | GAAGGTGAAGGTCGGAGT | SEQ ID NO: 3 |
| GAPDH Rv | GAAGATGGTGATGGGATTTC | SEQ ID NO: 4 |
| CD41 Fw | TGAGCCGCATTTACGTGGAAA | SEQ ID NO: 5 |
| CD41 Rv | CTTCACAGTAACGCTTGTCCC | SEQ ID NO: 6 |
| CD61 Fw | GTGACCTGAAGGAGAATCTGC | SEQ ID NO: 7 |
| CD61 Rv | TCACTCACTGGGAACTCGATG | SEQ ID NO: 8 |
| FLI-1 Fw | CCAACGAGAGGAGAGTCATCG | SEQ ID NO: 9 |
| FLI-1 Rv | TTCCGTGTTGTAGAGGGTGGT | SEQ ID NO: 10 |
| GATA1 Fw | TCACTCCCTGTCCCCAATAG | SEQ ID NO: 11 |
| GATA1 Rv | GGAGAGTTCCACGAAGCTTG | SEQ ID NO: 12 |
| NEF2 Fw | GCAGGAACAGGGTGATACAGC | SEQ ID NO: 13 |
| NEF2 Rv | GAGCAGGGGCAGTAAGTTGT | SEQ ID NO: 14 |
| SCL Fw | GGATGCCTTCCCTATGTTCA | SEQ ID NO: 15 |
| SCL Rv | GGTGTGGGGACCATCAGTAA | SEQ ID NO: 16 |

### Gene expression profile and data analysis

After 14 days, EBs from AND1 EV and AND1 SCL were ruptured and the total RNA was isolated using Trizol. The RNA quality was verified in Agilent Bioanalyzer 2100 platform. The RNA samples were labeled (Agilent Low Input Quick Labeling Kit Amp, Agilent Technologies) with Cy3 following the manufacturer's protocol. The hybridization method was performed using Agilent Gene Expression Hybridization kit (Agilent Technologies). The Cy3-labeled cRNA fragments were hybridized with Agilent Whole Human Genome Oligo Microarray 8x60K (Agilent Technologies) using Agilent's recommendations. Two independent samples were labeled and hybridized based on the condition thereof. Hierarchical clustering of the genes the samples was performed using correlation as distance metric. Putative candidate genes with a double change of >2 and a value of p<0.01 were considered differentially expressed (EV vs SCL). One hundred over-regulated and under-regulated genes were selected and analyzed using an online tool, Connectivity Map (Lamb, J. et al., 2006. Science 313(5795):1929-35.) (www. broadinstitute.org/cmap).

### Results

### The overexpression of SCL increases the production of megakaryocytes and platelets in hPSCs (hESCs and PSCs

The role of the SCL transcription factor in the hematopoietic specification of hPSCs has been elucidated in recent years. Human cell models were generated by forced expression of SCL in hPSCs (Yung, S. et al., 2011. Hum. Mol. Gene. 20(24):4932-46; Real, P.J. et al., 2012. Mol. Therapy 20(7): 1443-53). These cells conserve pluripotent properties and the increase in hematopoietic potential producing high levels of hematopoietic progenitor cells (CD34+ CD45+) and mature hematopoietic cells (CD45+). It has also been seen that overexpression accelerates the erythroid differentiation. Recently, different groups have demonstrated SCL participation in the differentiation of megakaryocytes from ESCs (Changraoui, H. et al., 2011. Blood 118(3):723-35) and from human hematopoietic stem cells CD34+ (Choi, E.S. et al., 1995. Blood 85(2):402-13.; Tao, H. et al., 1999. Exp. Hematol. 27(2):293-301; Ma, D.C. et al., 2000. Eur. J. Haematol. 64(5):304-14; De Bruyn, C. et al., 2005. Stem Cells Dev. 14(4):415-24). Nevertheless, the function of SCL during human hematopoiesis in embryos or the *in vitro* generation of megakaryocytes and platelets from hPSCs is unknown.

To determine the impact of the expression of SCL on both processes, hPSCs were differentiated in the presence of a set of human cytokines as already described by Dr. Lanza's group (Figure 1A). It consists of a 2-phase protocol. First, the hPSCs form embryonic bodies (EBs) immediately after 14 days. They break up on day 15 and the derived hPSCs are cultured with OP9 cells for 17 days in differentiation medium supplemented with heparin, TPO and SCF. The kinetics of emergence of megakaryocytes and platelets was quantified during the differentiation stage in co-culture using flow cytometry (Figure 1 B and C). The overexpression of SCL increased the production of megakaryocytes and platelets in two independent cell lines. As shown in Figure 1 C, after 18 days AND1 hPSCs (hESCs) overexpressing SCL (SCL) generated 15 times more megakaryocytes and 10 times more platelets than the control cells (EV). On day 20, EV and SCL showed the megakaryocyte and platelet production peak, with a production increase of 6 and 10 times, respectively, for cells overexpressing SCL. From day 24 onwards, megakaryopoiesis and thrombopoiesis decreased drastically in AND1 cells, the megakaryocyte differentiation process ending on day 28. In HS181 cells (hESCs), the kinetics of emergence of megakaryocytes and platelets is more progressive. Higher production of megakaryocytes and platelets was observed after 24 days with an increase of 10 and 3 times, respectively, in cells overexpressing SCL (SCL). This peak is followed by a sharp decrease until day 32, where the HS181 control cells (EV) completely cease the production of platelets and megakaryocytes, in turn, cells overexpressing SCL were still slightly productive. Finally, the same experiments were conducted in induced adult pluripotent cells (iPSCs) MUSH001 (Ross PJ et al., Stem Cells and Develop. 2010:19(8); 1221-1229). The data shown in Figure 1E shows that the overexpression of SCL increases the production of megakaryocytes and platelets like that occurring with hESCs. This data suggests that SCL positively regulates megakaryocyte differentiation from hPSCs, increases the number of megakaryocytes and platelets and maintains their production for a longer time period, without taking into account the hPSC line used.

On the other hand, which collection strategy is more effective in terms of megakaryocyte and platelet production has been studied. In another experiment, a fraction of cell culture supernatant was collected every 2-4 days for analysis and the equivalent volume was replenished with differentiation medium. The use of HS181 cells had a more progressive megakaryocyte differentiation, therefore three alternative plans were tested in parallel: i) one collection on day 22; ii) a single collection on day 26; a first collection on day 22 followed by medium replenishment and a second collection on day 26. Figure 1 D shows that double collection is the most effective method to obtain a better production of megakaryocytes and platelets, compared with single collections of days 22 and 26. These results indicate that the accumulative strategy is not beneficial for increasing the production of megakaryocytes and platelets, on the other hand, medium renewal favors the differentiation of hPSCs into megakaryocytes.

### The overexpression of SCL does not affect the morphological, molecular and functional properties of megakaryocytes and platelets derived from hPSCs

A detailed characterization at different levels was developed for the purpose of evaluating the impact of the overexpression of SCL on megakaryocytes and platelets derived from hPSCs. First, in HS181 megakaryocytes were purified from the EV and SCL supernatant during co-culture in different differentiation stages, early (20 days) and late (32 days). These cells were fixed with formaldehyde, concentrated with cytospin and characterized using staining with Papanicolaou solution (Figure 2A). As shown in Figure 2A, the HS181 SCL supernatant contained a higher concentration of megakaryocytes compared with the count of EV, however morphological differences were not detected between them. As expected, a more mature stage of differentiation was observed on day 32 determined because a larger number of large and multinucleated megakaryocytes were found. Secondly, an analysis of the gene expression at the start of the differentiation point (22 days) was performed using the HS181 control (EV) supernatant with the overexpression of SCL (SCL). Four transcription factors associated with megakaryocyte differentiation (*FLI-1, NFE2, GATA-1* and SCL) were found and two surface markers were analyzed using RT-PCR (Figure 2B). The expression of *FLI-1* and *NFE2* was over-regulated in HS181 SCL cells (SCL); whereas there were no differences in expression in *GATA-1, CD41* and *CD61* between the cells of the control (EV) and SCL.

Next, the functionality of hPSC-derived megakaryocytes was evaluated. Megakaryocytes derived from HS181 control culture (EV) and HS181 culture with the overexpression of SCL (SCL) on day 26 were purified and cultured on fibronectin-coated slides in the presence of human thrombin (2 U/mL) for 2 hours (Figure 2C). After this, unbound cells were eliminated and activated cells were washed twice with PBS, fixed with formaldehyde and stained using the Papanicolaou staining protocol. Curiously, both EV and SCL megakaryocytic cells were activated in a similar manner and had a similar number of bound, multinucleated cells (Figure 2C). Furthermore, from a supernatant fraction of HS181 control (EV) and HS181 with the overexpression of SCL, were collected on day 26, washed twice with PBS and activated by means of adding 5' adenine diphosphate (ADP) for 10 minutes. After staining the cells and platelets using the PAC-1 antibody recognizing the activated form of integrin αIIbβ3, they were analyzed using flow cytometry (Figure 2D). It is important to point out that the megakaryocytes and platelets produced from control cells (EV) and cells with the overexpression of SCL, are also functional and the ADP activation levels are very close. These results show that the overexpression of SCL does not modify the functionality of megakaryocytes and platelets derived from hPSCs.

### The overexpression of SCL accelerates the emergence of megakaryocyte progenitors from hPSCs

It has been proposed in earlier studies that the overexpression of SCL promoted the emergence of hematopoietic progenitors and erythro-megakaryocytes, as well as accelerate erythroids differentiation. However, megakaryocyte differentiation lineage has not been studied and this is the main subject of the study. To demonstrate that the overexpression of SCL promotes the emergence of megakaryocyte progenitors from hPSCs, the initial differentiation stage was extended to day 17 and the emergence of CD34 stem cells and megakaryocyte progenitors (CD34+CD41+) at different times (10,14 and 17 days) was analyzed using flow cytometry (Figure 3A and 3B). As shown in Figure 3C, the overexpression of SCL significantly accelerated the emergence of both populations. This effect is particularly drastic in the case of CD34+CD41+ populations which reached 5 to 7-8% of all the cells in EB overexpressing SCL during hPSC differentiation, in contrast with the low detection rate (below 0.5%) in control cells (EV). Furthermore, more mature derived megakaryocytes CD41+ CD42+ could be detected only on day 17 in EBs overexpressing SCL (data not shown).

For the purpose of further demonstrating the effect of the overexpression of SCL on the acceleration of the emergence of megakaryocyte progenitors, a gene expression analysis at the same time points (10, 14 and 17 days) was developed. The expression levels of transcription factors and surface markers associated with megakaryocytes with early differentiation were evaluated with quantitative RT-PCR using control EBs (EV) on day 10 (Figure 3D). It is important to point out that three transcription factors and two surface markers were significantly over-regulated in EBs overexpressing SCL at the three time points. The dynamics of expression of *NFE2, GATA-1, CD41* and *CD61* was very similar with the continuous increase of differentiation. While the expression of *FLI-1* was very high and constant throughout the entire EB SCL differentiation, a slight induction in the control EB (EV) was recorded. This data confirmed that the overexpression of SCL promotes and improves the emergence of megakaryocyte progenitors from hPSCs.

### The overexpression of SCL partially imitates the effect of histone deacetylase inhibitors during megakaryocyte differentiation

Once it was proven that the overexpression of SCL in hPSCs accelerated the emergence of megakaryocyte progenitors, the molecular and SCL-mediated transcription regulation mechanisms during megakaryocytic development from hPSCs was further examined in detail. Gene expression profiles were developed with oligonucleotide microarrays in control cells (EV) and cells with the overexpression of SCL (SCL) after 14 days of EB differentiation stage because it was the moment in which the EBs disaggregate and are seeded with stromal cells according to the standard protocol (Figure 1A). This analysis showed a more efficient megakaryocytic development on day 14 in EB SCL with many genes associated with hematopoietic differentiation and hemostasis. They include, among others, the five genes previously analyzed in Figure 3D. SCL-controlled transcriptional network regulating many megakaryocyte transcriptional regulators (FLI-1, GATA1, GATA2, NEF2, RUNX1, etc) and their target genes (data not shown) was also constructed using the Ingenuity software (www.ingenuity.com).

Furthermore, a public bioinformatic tool known as Connectivity Map, developed by Broad Institute, was used. This complex software provides a large amount of gene expression profiles from several standard cell lines treated with a list of bioactive molecules and compares these profiles with a particular expression data set. One hundred genes which are induced and repressed the most have been selected in a hierarchical clustering of EB EV vs EB SCL and the list of this analysis was shown. A very high correlation has been observed between the main genes and the expression profiles of several cell lines treated with Trichostatin A (TSA) at different concentrations (Table 2). The second compund which was also enriched was vorinostat or suberoylanilide hydroxamic acid. Both drugs are classified as histone deacetylase inhibitors. To experimentally validate this data *in silico,* a megakaryocyte differentiation assay was performed in the presence or absence of TSA at two alternative concentrations (Figure 4A) and megakaryocyte progenitors from wild-type hPSCs were analyzed. As shown in Figure 4B, the addition of TSA since the start of the differentiation process is associated with an increase in CD34+ CD41+ in the megakaryocyte progenitors after 14 days of the differentiation stage. Additional experiments will be conducted to optimize time, concentrations and alternative histone deacetylase inhibitors, such as valproic acid or SAHA which must be confirmed. In conclusion, these results show that the overexpression of SCL can control histone deacetylase activity during megakaryocyte differentiation and that the characterization of SCL-induced molecular pathways can aid in improving the process of developing megakaryocytes from hPSCs.

## Claims

1. *In vitro* use of a polynucleotide sequence consisting of SEQ ID No 1 or a nucleotide sequence with an identity of at least 80% with the sequence SEQ ID NO: 1, for producing functional platelet-producing megakaryocytes from stem cells, where said stem cells were not produced by methods using human embryos as raw material or destroying same.

2. *In vitro* use of a vector comprising the nucleotide sequence defined according to the preceding claim, for producing functional platelet-producing megakaryocytes from stem cells, where said stem cells were not produced by methods using human embryos as raw material or destroying same.

3. Use of the vector according to the preceding claim, where the vector is a lentiviral vector.

4. *In vitro* use of a gene construct comprising the vector as defined in any of claims 2-3, for producing functional platelet-producing megakaryocytes from stem cells, where said stem cells were not produced by methods using human embryos as raw material or destroying same.

5. *In vitro* use of the vector according to any of claims 2-3 or the gene construct according to claim 4, where the stem cells are selected from the list consisting of hematopoietic stem cells, pluripotent stem cells, induced pluripotent stem cells, embryonic stem cells, fibroblasts, induced megakaryocytes or any of the combinations thereof with the premise that said stem cells were not produced by methods using human embryos as raw material or destroying same.

6. Use of the vector or the gene construct according to claim 5, where the stem cells are pluripotent stem cells (PSCs).

7. Use of the vector or the gene construct according to any of claims 5-6, where the stem cells are cells from a mammal.

8. Use of the vector or the gene construct according to any of claims 5-7, where the stem cells are human cells.

9. A functional platelet-producing megakaryocyte produced by a method comprising:
a) contacting a vector as defined in any of claims 2-3 or a gene construct as defined in claim 4, with a stem cell, where said stem cell was not produced by methods using human embryos as raw material or destroying same;
b) selecting from step (a) stem cells which have been transduced or transformed with the vector and/or the gene construct; and
c) differentiating the stem cells of step (b) into mature megakaryocytes.

10. The megakaryocyte according to the preceding claim, where the stem cells are pluripotent stem cells (PSCs).

11. The megakaryocyte according to any of claims 9-10, where the stem cells are cells from a mammal.

12. The megakaryocyte according to any of claims 9-11, where the stem cells are human cells.

13. The megakaryocyte according to any of claims 9-12, where the differentiation of the stem cells into megakaryocytes of step (c) is carried out by differentiating the stem cells into megakaryocyte progenitors by means of adding a composition comprising a histone deacetylase inhibitor.

14. The megakaryocyte according to claim 13, where the histone deacetylase inhibitor is Trichostatin A.

15. The megakaryocyte according to any of claims 9-14, where the differentiation of the stem cells into mature megakaryocytes of step (c) comprises co-culturing the stem cells with stromal cells.

16. A method for the *in vitro* production of platelets which comprises culturing the functional platelet-producing megakaryocyte, as defined in any of claims 9-15, in a suitable culture medium.

17. Use of a megakaryocyte produced according to any of claims 9-15 or a platelet produced according to the method of claim 16, in the preparation of a medicinal product or as a vector directed at sites with vascular damage.

18. Use of a megakaryocyte produced according to any of claims 9-15 or a platelet produced according to the method of claim 16, in the preparation of a medicinal product for the treatment of platelet-related pathologies and/or acute megakaryoblastic leukemias.

19. Use of a histone deacetylase inhibitor for differentiating a stem cell into megakaryocyte, where said stem cell was not produced by methods using human embryos as raw material or destroying same.

20. Use of a composition comprising a histone deacetylase inhibitor for differentiating a stem cell into megakaryocyte, where said stem cell was not produced by methods using human embryos as raw material or destroying same.

21. A system or device for producing platelets for the *in vitro* and/or *ex vivo* production of the platelets of the invention comprising:
a) a bioreactor for the expansion of stem cells, where said stem cells were not produced by methods using human embryos as raw material or destroying same, in the presence of a first growth medium in fluid communication with;
b) a maturation chamber comprising stromal cells and/or an artificial bone marrow niche and a second growth medium, in which the maturation chamber is in fluid communication with;
c) a cell separation chamber for the selection of mature megakaryocytes which is in fluid communication with;
d) a platelet production module comprising a plurality of platelet production chambers, a three-dimensional matrix, a third growth medium, and a plurality of pumps for moving one third of the growth medium through the platelet production chambers, in which the platelet production module is in fluid communication with ;
e) a platelet collection chamber.
where the stem cells of step (a) are transfected with the polynucleotide sequence as defined in claim 1.


SEQUENCE LISTING

<110>  Fundación Pública Andaluza Progreso y Salud

<120>  Method for producing megakaryocytes and platelets

<130>  900552

<160>  16

<170>  PatentIn version 3.5

<210>  1
<211>  5018
<212>  DNA
<213>  Homo sapiens

<400>  1
gcccgggact atcccttcgc ggtgtagcgg cagccggaga cctggctgag gaggcaaccg      60

cgtagacacc tccctgctta gaaaacaaac actgaaccag accgatccca gttggagggt     120

tcgaaaatgt tccagacagc ctgtcgggag gggttgttgt tgctgttgga ctaaatagct     180

attcctgatt ggtcatgtat agggtttttt aaggcgggtg gggggaggag ggggtagagg     240

aaaggctcca aacacctgca ggttgggggc ggaaagctgt ttgcgattcc ctggactggt     300

tggtcgggga caggaggtaa ttcccagcca ttgaccccca tttctctctc tccctccctc     360

ttgccctgcc tctttctctc cacccctatc tttcctggaa actcgctttg ggcgcggcag     420

atcgcccagg accacaccgc agcgtaactg caggcctctc agcgaaaaag ggggaaagca     480

aagacccggg tgtgcatcct cttcctcggc ttccgcccct ttccggcgga gtggagatcc     540

tattcagagg ggccggtctc tctaaatatg ccccaggatg accgagcggc cgccgagcga     600

ggcggctcgc agtgaccccc agctagaggg acgggacgcg gccgaggcca gcatggcccc     660

cccgcacctg gtcctgctga acggcgtcgc caaggagacg agccgcgcgg ccgcagcgga     720

gcccccagtc atcgaactgg gcgcgcgcgg aggcccgggg ggcggccctg ccggtggggg     780

cggcgccgcg agagacttaa agggccgcga cgcggcgacg gccgaagcgc gccatcgggt     840

gcccaccacc gagctgtgca gacctcccgg gcccgccccg gcccccgcgc ccgcctcggt     900

tacagcggag ctgcccggcg acggccgcat ggtgcagctg agtcctcccg cgctggctgc     960

ccccgccgcc cccggccgcg cgctgctcta cagcctcagc cagccgctgg cctctctcgg    1020

cagcgggttc tttggggagc cggatgcctt ccctatgttc accaccaaca atcgagtgaa    1080

gaggagacct tccccctatg agatggagat tactgatggt ccccacacca aagttgtgcg    1140

gcgtatcttc accaacagcc gggagcgatg gcggcagcag aatgtgaacg gggcctttgc    1200

cgagctccgc aagctgatcc ccacacatcc cccggacaag aagctcagca agaatgagat    1260

cctccgcctg gccatgaagt atatcaactt cttggccaag ctgctcaatg accaggagga    1320

ggagggcacc cagcgggcca agactggcaa ggaccctgtg gtgggggctg gtgggggtgg    1380

aggtggggga gggggcggcg cgcccccaga tgacctcctg caagacgtgc tttcccccaa 1440

ctccagctgc ggcagctccc tggatggggc agccagcccg gacagctaca cggaggagcc 1500

cgcgcccaaa cacacggccc gcagcctcca tcctgccatg ctgcctgccg ccgatggagc 1560

cggccctcgg tgatgggtct gggccaccag gatcagccag gagggcgttc ttaggctgct 1620

gggatggtgg gcttcagggc aggtggggtg agaattgggc ggctctgaag caaggcggtg 1680

gacttgaact ttcctggatg tctgaacttt gggaagcctt tactgaccct ggggctggct 1740

tttctgtttc ctgtaccagt aggagatcag aaaaatggag caaagtggta ggtacttttt 1800

gtgaagacgg cacggtcttc cctcttccct cagtcccaaa tccttcccaa gtaagaggct 1860

ggagttgtca ctgcttttgg cctggagttt gggatccctg tctttcctaa gacctggggt 1920

tgtcagctct catctgaggc atccagcagt ctctgccttg cctttagccc ctcccaagct 1980

ggctggggtg gcctgtgtgg ccacttctgt ccatatttat aggtacccaa tagctgccca 2040

tttcgtgagc cccatcttca cccaggccta tgttgatcca tccagcttgc cagatgctgc 2100

agagtcacaa gcctcgaggt gccttcttca gggcctggtt gaagaagatg atcagtggac 2160

agtctgctct agatgagctg ggccggaggg tcaggaaacc cagtcgccct tacttcttgc 2220

cctggggatc aaagttctgc tttctcccca atgagacttg ccttcctaag cctgtggctg 2280

tggagacaat gtctgcagcc ctgagaaagc cctgtcgggc tttgtgtgaa ggcagagaaa 2340

gggacaatga tagtagagtg atatggagca agagatattt tgggcatgtg ggcttcaact 2400

cctcgacatc actgttcatg ctggcgagtg aatgccagtg tgctgatggg cgtacgctgg 2460

tgctgagtag atgcgcagcc ccatctgtgc attctcctgg atgcttagag ggatttcttt 2520

gctgtaagat gtctgtttgc tgatggtctg gtctatgttc cgaattgagc acaaaacctg 2580

tcctatgaat gctttgcatt tggaattttt gcttgacttc agttattggt ggaatcttta 2640

gcgctcaata ggaccaggat ccagcctcac ttctagggta tgggaaatcc aatcagagac 2700

caggccctgg ctaagaccca aacatatgca cattcactta gcagaacctt aaacacccct 2760

cagttgtgca gcttttggtc atcaagggtg cgtctgggag gttggtttaa tgcaatagaa 2820

gtgctcccct ctgaaagttg tacatgaaat ttttgtaaat cacatcctta tccttcatct 2880

tttaaagaaa taaccactgc aagtcctttt gtaaagtgaa gaatcctttt gtagaatgaa 2940

ccactgcccc ttcattgatt tcctgtgtca atccagatgg tgggatgtgg ttttcttaag 3000

gtgaggcctg tctgtgacct gcatctaagc ccatgggaca aattgcacag aagtcctgta 3060

tgtctgtcat tgtaccctta agtcacccta gccctctccc tctaggctct gccttcgagg 3120

tcagaggaga gatagcctgt ggccctgtcc tgccatgcaa gaactcatca ctgtggctgt 3180

ctggaaagcc cccccttata gtttgggctt cagcctagtg gcttgtcctc accatgatgg 3240

ggccctaatt cagccatgta cagacagaga atatgtctgc tcctttcccc ttccttttaa 3300

gtaaggtcca attctcgagc ttggggcaac attgttcacc tttgtagcac tcaggctctc 3360

cattcaattt caggctcccc agatcatgtt ttggtgaaaa ttagggttgg ttcctttcca 3420

acgtttggaa gatcctgtga ggagccccat ctgtctaaag atagagtcat tgctgtagga 3480

tctaaggctg tttgcttcac cgtggattcg cttgagttag gaatgagaag tagccacagt 3540

atggatgggt ggatgggttt tatgagatgg atcacatatt ttattaagaa ctcaaacttc 3600

tggctccctc ttctttcaga cttgccatgt gactctggct tggcctatct cctagggcta 3660

tggtgtggac tgaatgggat catgaaagta gacagttttg agaacgtaaa gaactttttc 3720

ttttccctca atctcaatcc tgcagtgggg tttcgcagcc tgagtccacg acctaggcag 3780

taggccggtg tgcctgactg cccagcattt gggtaattta gattgtaaac cgctttggcc 3840

tgagttattg agattgtcct catttctcca gattatctat ttgtgtgtgt gtgtgtgtgt 3900

gtgtgagaga cggtgtcttg ttctgtcact caggctggag tacagtggtg ccatcattgc 3960

tgtctgcagc cttgaactct gggctcaagc aatcctctca cctcagcctc ccgagtaggg 4020

aggaccacag gtgtgagcca ccacacctgg ctaattttta cttttttttt tttttggtag 4080

agatggagtc ttgctatatt gcccaggctg gtcttgaagt cctggcttca ggcaattctc 4140

ctgcctttgc ctccagaagc actgggatca caggtgtcag ccattgcacc cagcccagat 4200

tgtcttaatt tctatcttgt tccaaggcca gggacagtaa taagaatgga aaagagatat 4260

gggaacactg gcagactgtg taaaatgtaa tgcaactacc caaaacaagc ctggtaggaa 4320

agggcaagtc tttaggtctt tgtaagaact aaagaagatc tgtaattttt attttcaccc 4380

tctgtacccc atgaccttat ccttcctctc cttccttgtt acccatgaaa aactggcaac 4440

attccaagaa tagcatctgt acaaagggga aagaacataa aggtaaaaca aaacaaaaca 4500

acattttgag aacaaagatg accataacca ctgaagggaa tcacatcttt taagacaaat 4560

tcatattctt ttatttgtta tggcagatga caagatggta caacctttat tcttttccaa 4620

aataaaacaa agggcacagc atctgtagtc agccgacaac tatttcggcc ttttgggggt 4680

gggtctggcc gtacttgtga tttcgatggt acgtgaccct ctgctgaaga cttgccccct 4740

gcccgtgtac atagtgcatt gtttctgtgg gcgggcccag cactttccgt caacgttgta 4800

ctgtatgtga tgaattgcgt tggtctctgc atttttctgc agaagaggag taaccgctcc 4860

aggtaccttg acctttgtac agcccagagg ccaacactgt gggtgtgtga ctctttagca 4920

aaaaaaaccc atgtggtgat gatgtgtata tatatgtgag gatgtatcgg gaagatttct 4980

aaataaaagt tttacaaagg ggaaaaaaaa aaaaaaaa 5018

<210> 2
<211> 331

<212>  PRT
<213>  Homo sapiens

<400>  2

Met Thr Glu Arg Pro Pro Ser Glu Ala Ala Arg Ser Asp Pro Gln Leu
1               5                   10                  15

Glu Gly Arg Asp Ala Ala Glu Ala Ser Met Ala Pro Pro His Leu Val
            20                  25                  30

Leu Leu Asn Gly Val Ala Lys Glu Thr Ser Arg Ala Ala Ala Ala Glu
        35                  40                  45

Pro Pro Val Ile Glu Leu Gly Ala Arg Gly Gly Pro Gly Gly Gly Pro
    50                  55                  60

Ala Gly Gly Gly Gly Ala Ala Arg Asp Leu Lys Gly Arg Asp Ala Ala
65                  70                  75                  80

Thr Ala Glu Ala Arg His Arg Val Pro Thr Thr Glu Leu Cys Arg Pro
                85                  90                  95

Pro Gly Pro Ala Pro Ala Pro Ala Pro Ala Ser Val Thr Ala Glu Leu
            100                 105                 110

Pro Gly Asp Gly Arg Met Val Gln Leu Ser Pro Pro Ala Leu Ala Ala
        115                 120                 125

Pro Ala Ala Pro Gly Arg Ala Leu Leu Tyr Ser Leu Ser Gln Pro Leu
    130                 135                 140

Ala Ser Leu Gly Ser Gly Phe Phe Gly Glu Pro Asp Ala Phe Pro Met
145                 150                 155                 160

Phe Thr Thr Asn Asn Arg Val Lys Arg Arg Pro Ser Pro Tyr Glu Met
                165                 170                 175

Glu Ile Thr Asp Gly Pro His Thr Lys Val Val Arg Arg Ile Phe Thr
            180                 185                 190

Asn Ser Arg Glu Arg Trp Arg Gln Gln Asn Val Asn Gly Ala Phe Ala
        195                 200                 205

Glu Leu Arg Lys Leu Ile Pro Thr His Pro Pro Asp Lys Lys Leu Ser
    210                 215                 220

Lys Asn Glu Ile Leu Arg Leu Ala Met Lys Tyr Ile Asn Phe Leu Ala
225                 230                 235                 240

Lys Leu Leu Asn Asp Gln Glu Glu Glu Gly Thr Gln Arg Ala Lys Thr
                245                 250                 255

Gly Lys Asp Pro Val Val Gly Ala Gly Gly Gly Gly Gly Gly Gly Gly
            260                 265                 270

Gly Gly Ala Pro Pro Asp Asp Leu Leu Gln Asp Val Leu Ser Pro Asn
        275                 280                 285

Ser Ser Cys Gly Ser Ser Leu Asp Gly Ala Ala Ser Pro Asp Ser Tyr
    290                 295                 300

Thr Glu Glu Pro Ala Pro Lys His Thr Ala Arg Ser Leu His Pro Ala
305                 310                 315                 320

Met Leu Pro Ala Ala Asp Gly Ala Gly Pro Arg
                325                 330

<210>  3
<211>  18
<212>  DNA
<213>  Artificial Sequence

<220>
<223>  first GAPDH Fw

<400>  3
gaaggtgaag gtcggagt                                                    18

<210>  4
<211>  20
<212>  DNA
<213>  Artificial Sequence

<220>
<223>  first GAPDH Rv

<400>  4
gaagatggtg atgggatttc                                                  20

<210>  5
<211>  21
<212>  DNA
<213>  Artificial Sequence

<220>
<223>  first CD41 Fw

<400>  5
tgagccgcat ttacgtggaa a                                                21

<210>  6

<211>  21
<212>  DNA
<213>  Artificial Sequence

<220>
<223>  first CD41 Rv

<400>  6
cttcacagta acgcttgtcc c                                                21


<210>  7
<211>  21
<212>  DNA
<213>  Artificial Sequence

<220>
<223>  first CD61 Fw

<400>  7
gtgacctgaa ggagaatctg c                                                21


<210>  8
<211>  21
<212>  DNA
<213>  Artificial Sequence

<220>
<223>  first CD61 Rv

<400>  8
tcactcactg ggaactcgat g                                                21


<210>  9
<211>  21
<212>  DNA
<213>  Artificial Sequence

<220>
<223>  first FLI-1 Fw

<400>  9
ccaacgagag gagagtcatc g                                                21


<210>  10
<211>  21
<212>  DNA
<213>  Artificial Sequence

<220>
<223>  first FLI-1 Rv

<400>  10
ttccgtgttg tagagggtgg t                                                21


<210>  11
<211>  20
<212>  DNA
<213>  Artificial Sequence

<220>
<223>  first GATA1 Fw

<400>  11
tcactccctg tccccaatag                                                   20


<210>  12
<211>  20
<212>  DNA
<213>  Artificial Sequence

<220>
<223>  first GATA1 Rv

<400>  12
ggagagttcc acgaagcttg                                                   20


<210>  13
<211>  21
<212>  DNA
<213>  Artificial Sequence

<220>
<223>  first NEF2 Fw

<400>  13
gcaggaacag ggtgatacag c                                                 21


<210>  14
<211>  20
<212>  DNA
<213>  Artificial Sequence

<220>
<223>  first NEF2 Rv

<400>  14
gagcaggggc agtaagttgt                                                   20


<210>  15
<211>  20
<212>  DNA
<213>  Artificial Sequence

<220>
<223>  first SCL Fw

<400>  15
ggatgccttc cctatgttca                                                   20


<210>  16
<211>  20
<212>  DNA
<213>  Artificial Sequence

<220>
<223>  first SCL Rv

<400> 16
ggtgtgggga ccatcagtaa 20